# EUROPEAN PATENT APPLICATION

(11) **EP 2 962 723 A1**
(43) Date of publication of application: **06.01.2016**
(21) Application number: 14792020.1
(22) Date of filing: 10.02.2014
(51) Int. Cl.: A61M 39/10, A61M 25/00, F16L 11/12

(54) **MEDICAL TUBE, METHOD FOR MANUFACTURING SAME, AND TUBE CONNECTOR**

(30) Priority: 01.05.2013 JP 2013096459
(71) Applicant: Sumitomo Rubber Industries, Ltd., Kobe-shi, Hyogo 651-0072 (JP)
(72) Inventor: ISHIMARU, Takeshi, Kobe-shi Hyogo 651-0072 (JP)
(74) Representative: Manitz, Finsterwald & Partner GbR
(86) International application number: PCT/JP2014/053067
(87) International publication number: WO 2014/178211

(57) **Abstract**

A medical tube is provided, which has a detachment preventing function and provides a joint structure at lower costs without forming a step on an inner surface of the joint structure. The medical tube (1) is made of a chemical-resistant material, and includes a tube body (2) having a predetermined inner diameter (F) and a predetermined outer diameter (G), and an annular joint (3) provided at one end of the tube body (2) with respect to a longitudinal direction (X) of the tube body (2) integrally with the tube body (2), the annular joint (3) projecting outward from an outer peripheral surface (5) of the tube body (2) at an angle (D) smaller than a right angle, the annular joint (3) having a diameter progressively decreasing in a direction from a projection edge (8) toward the one end of the tube body (2).

## Description

### TECHNICAL FIELD

The present invention relates to a medical tube, and a medical tube production method. The present invention further relates to a tube connector for the medical tube.

### BACKGROUND ART

Medical tubes are used, for example, for feeding a liquid drug in a medical examination apparatus. Such a medical tube is connected to a device such as a pump or a solenoid valve through a joint (see, for example, PTL 1).

PTL1 discloses a joint which connects two cylindrical tubes having different diameters to each other. The joint has engagement holes provided in opposite end faces thereof to be engaged with outer peripheries of the corresponding tubes.

A conventional joint for a general purpose tube, e.g., a less expensive joint of so-called barb fitting type, is used for a medical tube. The joint of this type includes a tubular member to be engaged with an inner periphery of a cylindrical tube, and an annular projection circumferentially provided on an outer peripheral surface of the tubular member. Depending on an internal pressure to be applied to the tube, as required, an outer periphery of the tube is clamped with a ring band for prevention of detachment of the tube.

Other conventional general-purpose joints are proposed in PTL2 and PTL3. For prevention of detachment of a cylindrical tube, for example, the joint of PTL2 includes an inner cylinder to be engaged with an inner periphery of the tube, and an outer cylinder to be engaged with an outer periphery of the tube.

### CITATION LIST

### PATENT LITERATURE

PTL1: JP2011-212174A
PTL2: JP2012-47233A
PTL3: JP2011-220469A

### SUMMARY OF INVENTION

### TECHNICAL PROBLEM

Where the joint of the barb fitting type is employed for the medical tube, however, the liquid drug or a sample is liable to stagnate between the inner surface of the tube and the joint because the joint is located inside the tube. Therefore, it is necessary to clean the joint after the liquid drug is used.

This problem also arises when the joint of PTL2 is employed for the medical tube.

On the other hand, the joint of PTL1 is free from the possibility of the stagnation of the liquid drug because the joint is located outside the tube. However, the tube is liable to be detached from the joint without an arrangement for the prevention of the tube detachment.

In view of the foregoing, it is a principal object of the present invention to provide a medical tube which is capable of providing a joint structure at lower costs while preventing the stagnation of the liquid drug inside the joint structure and has a detachment preventing function, and to provide a method for producing the medical tube. It is another object of the present invention to provide a tube connector to be connected to the medical tube.

### SOLUTION TO PROBLEM

According to an inventive aspect of claim 1, there is provided a medical tube including: a tube body made of a chemical-resistant material and having a predetermined inner diameter and a predetermined outer diameter; and an annular joint provided integrally with the tube body at least at one of opposite ends of the tube body with respect to a longitudinal direction of the tube body, the annular joint projecting outward from an outer peripheral surface of the tube body at an angle not greater than a right angle, the annular joint having a wedge-like sectional shape and having a diameter progressively decreasing in a direction from a projection edge toward the one end of the tube body.

According to an inventive aspect of claim 2, the medical tube of claim 1 is used in a medical examination apparatus using a liquid drug.

According to an inventive aspect of claim 3, there is provided a method for producing the medical tube of claim 1 or 2, the method including the steps of: preparing a tube body; placing an annular joint material piece around an outer periphery of the tube body, the annular joint material piece being made of the same material as the tube body; inserting a rod-shaped core die into the tube body from one of opposite ends of the tube body, the core die having a diameter that is equal to the inner diameter of the tube body; and holding and heating the core die, the tube body and the annular joint material piece between a pair of mold halves.

According to an inventive aspect of claim 4, there is provided a tube connector to be engaged with the medical tube of claim 1 or 2, the tube connector including: a tube insertion port having an inner diameter that is smaller than the outer diameter of the one end of the medical tube and permits forcible insertion of the one end of the medical tube; a first cavity extending inward from the tube insertion port to a predetermined depth and having the same inner diameter as the tube insertion port; a second cavity provided inward of the first cavity as continuously extending from the first cavity coaxially with the first cavity, the second cavity expanding generally radially outward from the first cavity at an angle not greater than a right angle, the second cavity having a greater inner diameter than the first cavity, the second cavity being configured so that the annular joint of the medical tube can be fitted in the second cavity; and a third cavity provided inward of the second cavity as extending continuously from the second cavity coaxially with the first and second cavities, and having the same inner diameter as the medical tube.

### ADVANTAGEOUS EFFECTS OF INVENTION

According to the inventive aspect of claim 1, the annular joint has a wedge-like sectional shape. Therefore, where the medical tube is used to provide the joint structure, for example, the annular joint functions as a detachment preventing member with the medical tube inserted in a connection hole of the tube connector. Thus, the stagnation of the liquid drug can be prevented without the need for providing a detachment preventing member (which is required in the case of the conventional joint) inside the medical tube. As a result, the inside of the medical tube can be easily cleaned after use. Since the medical tube has the detachment preventing function, the joint structure can be simplified. This reduces the total production costs of the medical tube and the joint.

According to the inventive aspect of claim 2, the stagnation of the liquid drug and the sample to be used in the medical examination apparatus can be prevented. Therefore, the influence of the remaining liquid drug on the medical examination is eliminated. As a result, the medical examination apparatus can perform the examination with an improved accuracy.

According to the inventive aspect of claim 3, it is possible to produce the medical tube integrally including the tube body and the annular joint while preventing formation of a step on an inner surface of the tube body. Further, the formation of the annular joint and the connection of the annular joint to the tube body can be simultaneously achieved. In addition, a less expensive existing product can be used as the tube body. This reduces the production costs.

According to the inventive aspect of claim 4, the tube body of the medical tube is located in the first cavity and the annular joint of the medical tube is located in the second cavity, whereby the medical tube can be connected to the tube connector in a detachment prevented state. The inside of the medical tube and the third cavity of the tube connector can be connected to each other with no step, thereby preventing the stagnation of the liquid drug.

### BRIEF DESCRIPTION OF DRAWINGS

FIG. 1 is a sectional view of a medical tube 1 according to an embodiment of the present invention.
FIG. 2 is a sectional view of a tube connector 11 to which the medical tube 1 can be connected.
FIGS. 3(a) to 3(d) are schematic diagrams for explaining the steps of a method for producing the medical tube 1, particularly, FIG. 3(a) being a side view of a tube body 2 to be used as a material for the medical tube 1, FIG. 3(b) being a sectional view illustrating the tube body 2 and a mold 27, 28, 29, FIG. 3(c) being a sectional view illustrating the tube body 2 and the mold 27, 28, 29 in a molding step, FIG. 3(d) being a sectional view illustrating the medical tube 1 in a complete state.
FIG. 4 is a sectional view of a tube connector 11A according to a modification of the embodiment.

### DESCRIPTION OF EMBODIMENTS

Referring to the attached drawings, embodiments of the present invention will hereinafter be described in detail.

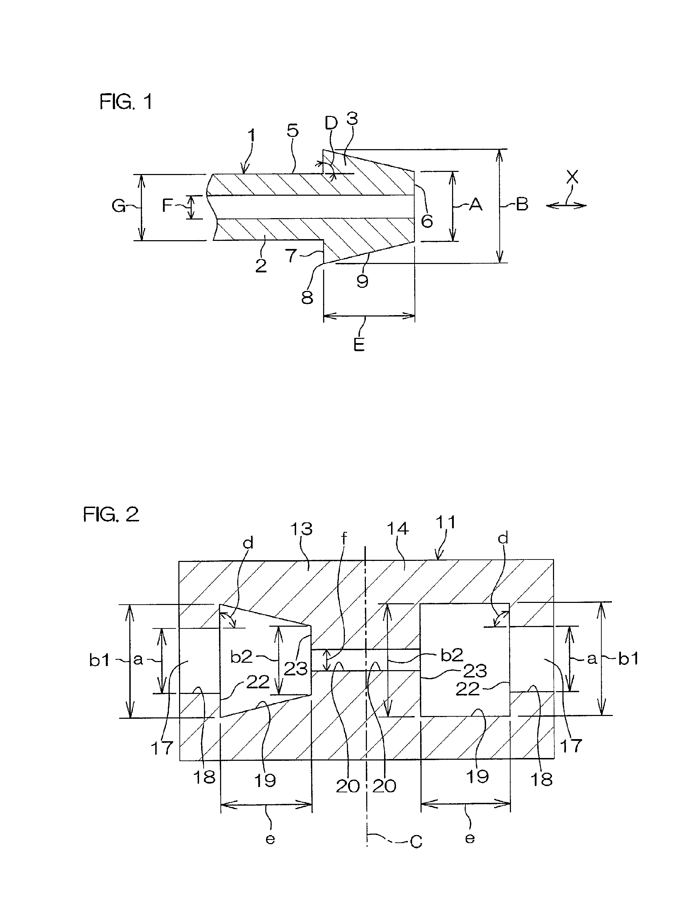

FIG. 1 is a sectional view illustrating one end portion of a medical tube 1 according to an embodiment of the present invention.

Referring to FIG. 1, the medical tube 1 includes a tube body 2, and an annular joint 3 provided integrally with the tube body 2 at one end of the tube body 2 with respect to a longitudinal direction X. In FIG. 1, only the one end of the medical tube 1 is shown. The annular joint 3 may be provided only at the one end of the tube body 2 with respect to the longitudinal direction X, but annular joints 3 may be respectively provided at opposite ends of the tube body 2.

The tube body 2 has an elongated cylindrical shape, and has a predetermined inner diameter F and a predetermined outer diameter G. The tube body 2 is made of a chemical-resistant material. Examples of the chemical-resistant material include thermoplastic olefin resin materials and thermoplastic urethane resins.

The annular joint 3 is an annular projection projecting radially outward from an outer peripheral surface 5 of the tube body 2. The annular joint 3 endlessly continuously extends circumferentially on the outer periphery of the tube body 2. The annular joint 3 may have circumferential ends. Hereinafter, the medical tube 1 will be described assuming that the annular joint 3 is endless.

The annular joint 3 has an annular engagement surface 7 which extends outward at a generally right angle D from the outer peripheral surface 5 of the tube body 2 for prevention of tube detachment. The engagement surface 7 has an outer periphery which defines a projection edge 8. The annular joint 3 has a guide tilt surface 9 having a diameter progressively decreasing in a direction from the projection edge 8 toward a distal end 6 of the tube body 2 at the one end. The guide tilt surface 9 defines an outer peripheral surface of the annular joint 3, and extends to the distal end 6 of the medical tube 1. The annular joint 3 has a wedge-like shape in section taken across the circumference thereof.

The angle D at which the engagement surface 7 of the annular joint 3 extends outward from the outer peripheral surface 5 of the tube body 2 may be a right angle, an angle smaller than the right angle, or an angle close to the right angle.

The annular joint 3 and the tube body 2 are made of the same material, but may be made of different materials. The medical tube 1 will be further described assuming that the tube body 2 and the annular joint 3 are made of the same material.

FIG. 2 is a sectional view of a tube connector 11. Referring to FIGS. 1 and 2, the tube connector 11 connects one-side ends of two medical tubes 1 to each other in opposed relation.

The tube connector 11 includes a first connector portion 13 to which the one end of one of the medical tubes 1 is connected so that a liquid drug (liquid) can flow through the tube connector 11, and a second connector portion 14 to which the one end of the other medical tube 1 is connected so that the liquid drug (liquid) can flow through the tube connector 11.

A left half of the tube connector 11 (e.g., a portion of the tube connector 11 shown on the left side of a one-dot-and-dash line C) serves as the first connector portion 13, and a right half of the tube connector 11 (e.g., a portion of the tube connector 11 shown on the right side of the one-dot-and-dash line C) serves as the second connector portion 14. The tube connector 11 is constructed so as to permit communication of the liquid drug between the first connector portion 13 and the second connector portion 14.

The first connector portion 13 and the second connector portion 14 are integrally formed of the same material. For example, the tube connector 11 is molded from a resin such as PBT (polybutylene terephthalate), PPS (polyphenylene sulfide) or PEEK (polyethylene ether ketone) by an injection molding method.

The first connector portion 13 and the second connector portion 14 have substantially the same construction. For convenience of description, the first connector portion 13 and the second connector portion 14 herein have a difference, but may have the identical construction.

The first connector portion 13 and the annular joint 3 at the one end of the corresponding medical tube 1 define a joint structure. The first connector portion 13 and the annular joint 3 at the one end of the medical tube 1 cooperatively function as a detachment preventing member. The first connector portion 13 is detachable from the one end of the medical tube 1.

The first connector portion 13 includes a tube insertion port 17 provided in a surface thereof, a first cavity 18 extending inward from the tube insertion port 17, a second cavity 19 provided inward of the first cavity 18 and having a greater diameter than the first cavity 18, and a third cavity 20 provided continuously inward of the second cavity 19 to define at least a part of a connection flow passage.

The tube insertion port 17 has an inner diameter (value (a)) that is smaller than the outer diameter (minimum value A) of the one end of the medical tube 1 and permits forcible insertion of the one end of the medical tube 1. The diameter (a) of the tube insertion port 17 is preferably smaller than the diameter A of the distal end 6 of the medical tube 1 and not smaller than 95 % of the diameter A. More specifically, the diameter (a) has a value that is equal to 95 % of the diameter A of the distal end 6 of the medical tube 1.

The first cavity 18 extends straight inward from the tube insertion port 17 to a predetermined depth, and has the same inner diameter as the tube insertion port 17.

The second cavity 19 is provided inward of the first cavity 18, and continuously extends from the first cavity 18 coaxially with the first cavity 18. The second cavity 19 expands outward at an angle d not greater than a right angle from the first cavity 18, and has inner diameters b1 and b2 that are greater than the inner diameter of the first cavity 18. Thus, the second cavity 19 is configured so that the annular joint 3 of the medical tube 1 can be fitted in the second cavity 19. The second cavity 19 extends inward to a predetermined depth e with its diameter gradiently decreasing in an inward direction.

The inner diameter b1 of the second cavity 19 is the inner diameter of a portion of the second cavity 19 adjacent to the first cavity 18, and has a value that is not smaller than the outer diameter B of the projection edge 8 of the annular joint 3. The inward inner diameter b2 has a value that is not smaller than the outer diameter A of the distal end 6 of the annular joint 3. The depth e of the second cavity 19 as measured axially is equal to the length E of the annular joint 3 of the medical tube 1.

The third cavity 20 is provided inward of the second cavity 19, and extends continuously from the second cavity 19 coaxially with the first cavity 18 and the second cavity 19. The third cavity 20 has an inner diameter f that is equal to the inner diameter F of the medical tube 1.

The second connector portion 14 has substantially the same construction as the first connector portion 13 with a difference from the first connector portion 13 in that the second cavity 19 has a rectangular shape in section taken along a plane including the axis of the second cavity 19. Components of the second connector portion 14 will be designated by the same reference characters as those of the first connector portion 13, and duplicate detailed description will be omitted.

In the second cavity 19 of the second connector portion 14, the diameter b1 of a portion adjacent to the first cavity 18 and the diameter b2 of a portion adjacent to the third cavity 20 have the same value. These diameters b1, b2 have the same value as the maximum diameter B of the annular joint 3, or have a value greater than the maximum diameter B of the annular joint 3.

Though not shown, the diameters b1, b2 of the second cavity 19 may have different values. The diameter b1 may have the same value as the maximum diameter B of the annular joint 3 or have a value greater than the maximum diameter B. The diameter b2 may have the same value as the maximum diameter B of the annular joint 3 or have a value greater than the maximum value B.

How to connect the medical tube 1 to the first connector portion 13 of the tube connector 11 will be described by way of example.

The one end of the medical tube 1 is inserted into the tube insertion port 17 of the first connector portion 13. The medical tube 1 is squeezed into the first connector portion 13, while the one end of the medical tube 1 and the first connector portion 13 are resiliently deformed. Thus, the tube body 2 of the medical tube 1 is located in the first cavity 18, and the annular joint 3 of the medical tube 1 is located in the second cavity 19.

In this state, the engagement surface 7 of the annular joint 3 of the medical tube 1 and an end wall 22 of the second cavity 19 are engaged with each other to function as the detachment preventing member. The end face of the distal end 6 of the tube body 2 of the medical tube 1 abuts against an end wall 23 of the second cavity 19, whereby the inside of the tube body 2 communicates with the third cavity 20.

Thus, the medical tube 1 can be connected to the first connector portion 13 in a detachment prevented state. Further, the inside of the medical tube 1 and the third cavity 20 of the first connector portion 13 can be connected to each other with no step, thereby preventing the stagnation of the liquid drug.

The medical tube 1 can be removed from the first connector portion 13 by pulling the medical tube 1 out of the connector by a strong force while reducing the diameter of the annular joint 3.

A valve or a stopper (not shown) for opening and closing the third cavity 20 serving as the internal flow passage may be provided between the first connector portion 13 and the second connector portion 14. Further, the first connector portion 13 (or the second connector portion 14) may be provided at one end of a flow passage of a device such as a pump for connection to the medical tube 1. Therefore, at least one connector portion 13, 14 is required for the arrangement for the connection to the medical tube 1.

As described above, the annular joint 3 provided at the one end of the medical tube 1 has a wedge-like sectional shape. Therefore, when the medical tube 1 is used to provide a joint structure, for example, the medical tube 1 is inserted into the tube insertion port 17 of the tube connector 11 and, in this state, the annular joint 3 functions as the detachment preventing member. Therefore, the stagnation of the liquid drug can be prevented without the need for providing the detachment preventing member (which is required in the case of the conventional joint) inside the medical tube 1. As a result, the inside of the medical tube 1 can be easily cleaned after use. Since the medical tube has the detachment preventing function, the joint structure (connector structure) can be simplified. This reduces the total production costs of the medical tube 1 and the joint.

Though not shown, the medical tube 1 and the connector portions 13, 14 are preferably used in a medical examination apparatus employing a liquid drug. The medical examination apparatus includes a liquid drug storing container, a liquid drug feeding pump, a solenoid valve for controlling the flow of the liquid drug, and an examination device which performs a predetermined examination on the liquid drug. For communication of the liquid drug between these devices, the medical tube 1 is used. Further, the first connector portion 13 (or the second connector portion 14) may be used for connection between the one end of the medical tube 1 and the device such as the pump or the solenoid valve.

In this case, the stagnation of the liquid drug and a sample to be used in the medical examination apparatus can be prevented, so that the influence of the remaining liquid drug on the examination is eliminated. As a result, the medical examination apparatus can perform the examination with an improved accuracy. The medical tube 1 may be used in an application field other than the medical examination apparatus.

The medical tube 1 may be produced, for example, by the following production method.

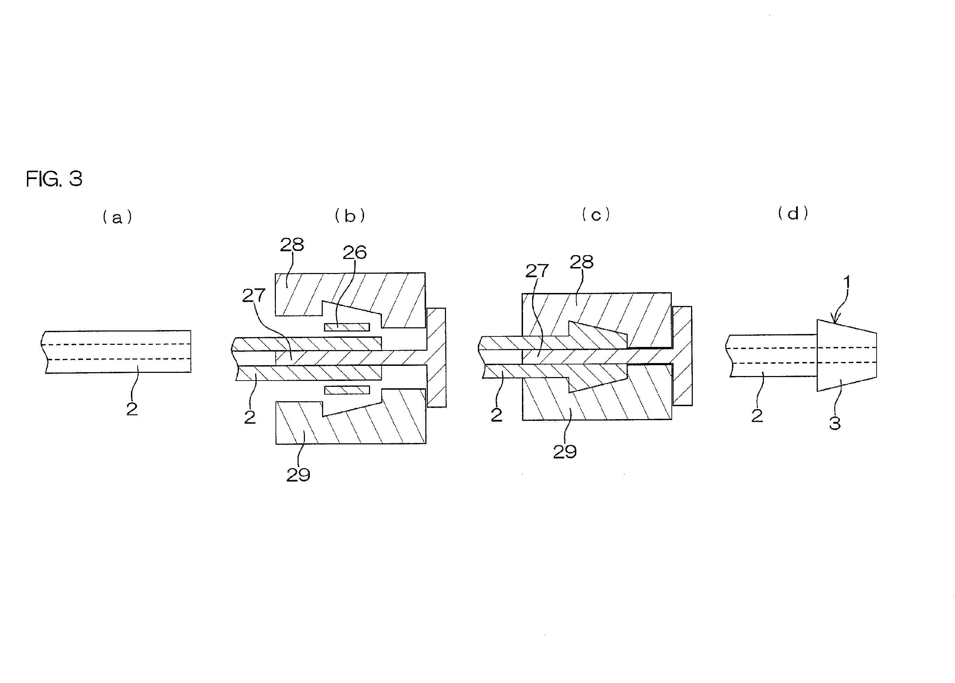

FIGS. 3(a) to 3(d) are schematic diagrams for explaining the steps of the method for producing the medical tube 1. FIG. 3 (a) is a side view of a tube body 2 to be used as a material for the medical tube 1. FIG. 3(b) is a sectional view illustrating the tube body 2 and a mold. FIG. 3(c) is a sectional view illustrating the tube body 2 and the mold in a molding step. FIG. 3(d) is a sectional view illustrating the medical tube 1 in a complete state.

The production method for the medical tube 1 includes the following steps.

Referring to FIG. 3(a), the tube body 2 is prepared in the first step.

Referring to FIG. 3(b), a material piece 26 for the annular joint 3 (made of the same material as the tube body 2) is placed around one end portion of the prepared tube body 2 in the second step. The material piece 26 may be a sheet member or an annular member surrounding the tube body 2. The material piece 26 may be bonded to an outer periphery of the one end portion of the tube body 2.

In the third step, the tube body 2 with the material piece 26 placed around the tube body 2 is set in a mold. The mold to be used includes a core die 27 and a pair of mold halves 28, 29. More specifically, a rod-shaped core die 27 having the same diameter as the inner diameter of the tube body 2 is inserted from one end of the tube body 2 into the tube body 2 with the material piece 26 placed around the tube body 2.

Referring to FIG. 3(c), the core die 27, the tube body 2 and the material piece 26 for the annular joint 3 are held between the pair of mold halves 28 and 29, and heated in the fourth step. At this time, a predetermined amount of the material for the tube body 2 may be additionally fed into a mold space defined between the mold halves 28, 29 for formation of the annular joint 3. The material piece 26 for the annular joint 3 may be heat-bonded to the outer periphery of the tube body 2. If this is difficult, an adhesive agent may be used. Where the tube body 2 is made of a crosslinked TPE (thermoplastic elastomer) and the material piece 26 for the annular joint 3 is made of an uncrosslinked TPE, for example, the material piece 26 can be heat-bonded to the tube body 2. This arrangement may also be employed where a crosslinked rubber dispersed TPE (DC material) is used instead of the TPE. Where a silicone resin or a PTFE resin is used instead of the TPE, an adhesive agent may be required.

In the fourth step, the tube body 2 and the material piece 26 for the annular joint 3 are unified and, at the same time, the annular joint 3 is formed into a predetermined shape. Thus, the medical tube 2 is completed.

Referring to FIG. 3(d), the completed medical tube 1 is demolded from the mold halves 28, 29.

This production method makes it possible to produce the medical tube 1 which integrally includes the tube body 2 and the annular joint 3 without formation of a step on the inner surface of the tube body 2. Further, the formation of the annular joint 3 and the bonding between the annular joint 3 and the tube body 2 can be simultaneously achieved. In addition, a less expensive existing tube product, for example, can be used as the tube body 2, thereby reducing the production costs.

The second step and the third step may be exchanged. That is, the core die 27 may be inserted into the tube body 2 in the second step, and the material piece 26 for the annular joint 3 may be placed around the tube body 2 fitted around the core die 27 in the third step.

The aforementioned embodiment may be modified in the following manner. The modification of the embodiment has substantially the same construction as the embodiment with some differences, which will be mainly described below.

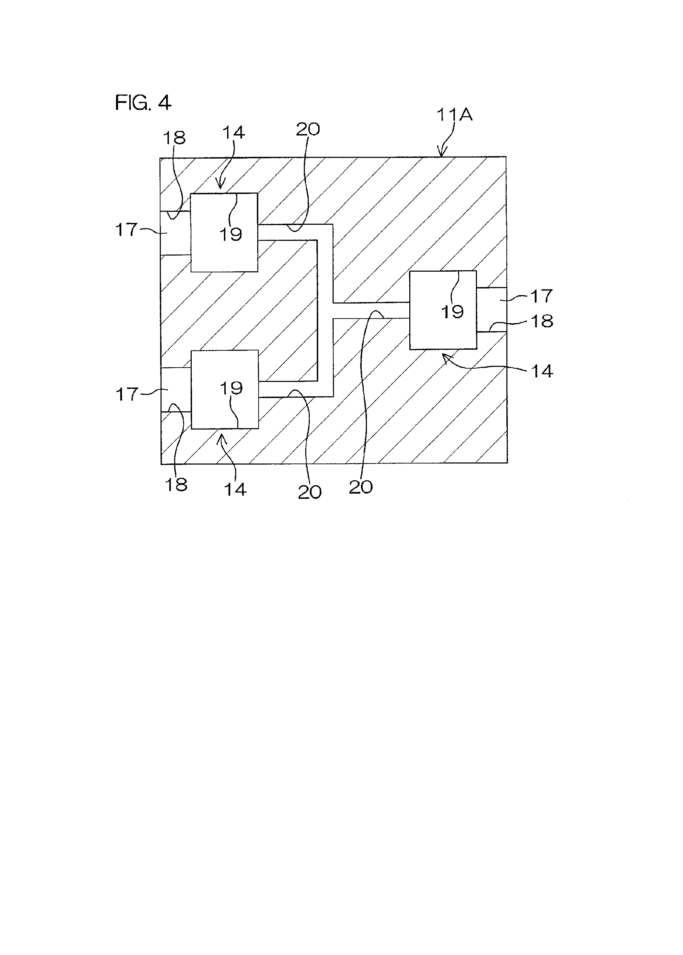

FIG. 4 is a sectional view of a tube connector 11A according to the modification.

The tube connector 11A has three connector portions 14 for connecting three medical tubes 1 to each other. A liquid drug can flow between these connector portions 14. That is, the third cavities 20 of the respective connector portions 14 communicate with each other. At least one of the three connector portions 14 of the tube connector 11A may be replaced with a connector portion 13. Further, the tube connector 11A may include four or more connector portions 13, 14. In these tube connectors according to this modification, a valve and/or a stopper may be provided.

It should be understood that various design modifications may be made within the scope of the present invention defined by the appended claims.

### REFERENCE SIGNS LIST

- 1: MEDICAL TUBE
- 2: TUBE BODY
- 3: ANNULAR JOINT
- 5: OUTER PERIPHERAL SURFACE
- 8: PROJECTION EDGE
- 11, 11A, 13, 14: TUBE CONNECTOR (CONNECTOR PORTION)
- 17: TUBE INSERTION PORT
- 18: FIRST CAVITY
- 19: SECOND CAVITY
- 20: THIRD CAVITY
- 26: MATERIAL PIECE
- 27: CORE DIE
- 28, 29: MOLD HALVES
- (a): INNER DIAMETER OF TUBE INSERTION PORT
- b1, b2: INNER DIAMETER OF SECOND CAVITY
- D: ANGLE
- F: INNER DIAMETER OF TUBE BODY
- G: OUTER DIAMETER OF TUBE BODY
- f: INNER DIAMETER OF THIRD CAVITY
- X: LONGITUDINAL DIRECTION

## Claims

1. A medical tube comprising:
a tube body made of a chemical-resistant material and having a predetermined inner diameter and a predetermined outer diameter; and
an annular joint provided integrally with the tube body at least at one of opposite ends of the tube body with respect to a longitudinal direction of the tube body, the annular joint projecting outward from an outer peripheral surface of the tube body at an angle not greater than a right angle, the annular joint having a wedge-like sectional shape and having a diameter progressively decreasing in a direction from a projection edge toward the one end of the tube body.

2. The medical tube according to claim 1, which is used in a medical examination apparatus using a liquid drug.

3. A method for producing the medical tube according to claim 1 or 2, the method comprising the steps of:
preparing a tube body;
placing an annular joint material piece around an outer periphery of the tube body, the annular joint material piece being made of the same material as the tube body;
inserting a rod-shaped core die into the tube body from one of opposite ends of the tube body, the core die having a diameter that is equal to an inner diameter of the tube body; and
holding and heating the core die, the tube body and the annular joint material piece between a pair of mold halves.

4. A tube connector to be engaged with the medical tube according to claim 1 or 2, the tube connector comprising:
a tube insertion port having an inner diameter that is smaller than an outer diameter of the one end of the medical tube and permits forcible insertion of the one end of the medical tube;
a first cavity extending inward from the tube insertion port to a predetermined depth and having the same inner diameter as the tube insertion port;
a second cavity provided inward of the first cavity as continuously extending from the first cavity coaxially with the first cavity, the second cavity expanding generally radially outward from the first cavity at an angle not greater than a right angle, the second cavity having a greater inner diameter than the first cavity, the second cavity being configured so that the annular joint of the medical tube can be fitted in the second cavity; and
a third cavity provided inward of the second cavity as extending continuously from the second cavity coaxially with the first and second cavities, and having the same inner diameter as the medical tube.
